(19) Europäisches Patentamt
European Patent Office
Office européen des brevets

(11) Numéro de publication : **0 135 638**
**B1**

# (12) FASCICULE DE BREVET EUROPÉEN

(45) Date de publication du fascicule du brevet : **12.11.86**

(51) Int. Cl.⁴ : **C 25 B 3/02**

(21) Numéro de dépôt : **83401243.7**

(22) Date de dépôt : **16.06.83**

(54) **Procédé électrochimique de préparation de sulfoxydes de dérivés de thioformamides utiles comme médicaments.**

(43) Date de publication de la demande :
**03.04.85 Bulletin 85/14**

(45) Mention de la délivrance du brevet :
**12.11.86 Bulletin 86/46**

(84) Etats contractants désignés :
**AT BE CH DE FR GB IT LI LU NL SE**

(56) Documents cités :
**DE-A- 1 922 721**
**US-A- 4 297 181**

(73) Titulaire : **RHONE-POULENC SANTE**
**Les Miroirs 18 Avenue d'Alsace**
**F-92400 Courbevoie Cedex (FR)**

(72) Inventeur : **Bizot, Jean**
**41, rue des Champs**
**F-91420 Morangis (FR)**
Inventeur : **Deprez, Dominique**
**68, route de Marcoussis**
**F-91310 Montlhery (FR)**

(74) Mandataire : **Le Goff, Yves et al**
**RHONE-POULENC RECHERCHES Brevets Pharma**
**25, Quai Paul Doumer**
**F-92408 Courbevoie (FR)**

**EP 0 135 638 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation de sulfoxydes de dérivés de la thioformamide de formule générale :

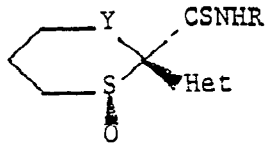

(I)

Dans la formule générale (I), R représente un atome d'hydrogène ou un radical alcoyle contenant 1 à 4 atomes de carbone en chaîne droite ou ramifiée, Het représente un radical hétérocyclique à caractère aromatique contenant un ou deux atomes d'azote choisi parmi pyridyle-3 (éventuellement substitué par un radical alcoyle contenant 1 à 4 atomes de carbone en chaîne droite ou ramifiée ou par un atome d'halogène), quinolyle-3, pyridazinyle-4, pyrimidinyle-5, thiazolyle-5, thiéno [2,3-b] pyridyle-5 et thiéno [3,2-b] pyridyle-6 et Y représente une liaison de valence ou un radical méthylène.

La présence d'un atome d'oxygène sur le soufre crée dans la molécule une dissymétrie qui, associée au carbone asymétrique voisin, conduit théoriquement à 4 stéréoisomères possibles, éventuellement séparables en deux couples racémiques. La présente invention concerne un procédé sélectif de préparation des produits de formule générale (I) c'est-à-dire dans laquelle le sulfoxyde se trouve en position trans par rapport au groupement thioamide.

Selon l'invention, les produits de formule générale (I) peuvent être obtenus par oxydation électrochimique des produits de formule générale :

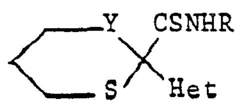

(II)

dans laquelle Het, R et Y sont définis comme précédemment, en opérant dans un électrolyte à forte teneur en eau, à un pH compris entre 7 et 7,5 et en présence d'un agent spécifique d'oxydation $X^+$ obtenu in situ par voie électrochimique à partir d'un halogénure $X^-$ et en travaillant à un potentiel d'électrode imposé voisin du potentiel d'oxydation de $X^-$.

Dans la pratique, il est particulièrement avantageux pour donner naissance à l'agent d'oxydation $X^+$ d'utiliser un iodure de métal alcalin tel que l'iodure de potassium ou un halogénure d'ammonium tel que l'iodure d'ammonium ou l'iodure de triéthyl n-propylammonium ou le bromure de tétraéthylammonium, ou encore un iodure d'aryle tel que l'iodure de phényle, en opérant à un potentiel d'électrode imposé voisin du potentiel d'oxydation de l'iodure (0,6 à 0,8 V par rapport à une électrode de référence au calomel saturée).

L'électrolyte dans lequel se fait la réaction est constitué généralement :

— d'un solvant organique miscible à l'eau permettant de dissoudre la substance à oxyder de formule générale (II), tel que l'acétonitrile ou un alcool comme le méthanol ou l'éthanol,

— d'eau distillée ou permutée,

— d'une solution tampon à pH 7 dans l'eau généralement constituée par un mélange d'une solution aqueuse 0,1 M d'hydrogénophosphate d'ammonium et de dihydrogénophosphate d'ammonium.

Les proportions relatives d'eau et de solvant organique dépendent de la solubilité dans l'eau du sulfure de formule générale (II) à oxyder. Le pourcentage total d'eau dans l'électrolyte peut varier entre 10 et 99 ; il est généralement compris entre 40 et 80 %.

La quantité d'électricité nécessaire en pratique pour effectuer l'oxydation d'un produit de formule générale (II) en un produit de formule générale (I) est de 4 à 8 Faradays par mole.

De préférence, l'oxydation s'effectue dans un électrolyseur à diaphragme en opérant à une température comprise entre 0 °C et la température de reflux du mélange réactionnel, de préférence entre 20 et 60 °C.

Selon une mise en œuvre préférée du procédé, l'oxydation électrolytique est effectuée dans un électrolyseur comportant une anode et une électrode de référence, un compartiment anodique, un diaphragme séparateur, un compartiment cathodique et une cathode dont les caractéristiques sont les suivantes :

a) L'anode est un solide inattaquable au potentiel auquel s'effectue la réaction et constituée d'un matériau conducteur de l'électricité, de préférence le platine sur lequel l'oxydation du produit de formule générale (II) a lieu à un potentiel inférieur au potentiel d'oxydation des constituants du solvant ; ce potentiel est mesuré par rapport à une électrode de référence au calomel saturée séparée de l'électrolyte par un gel d'agar-agar avec KCl.

b) Le compartiment anodique contient l'électrolyte indiqué précédemment et le produit de formule

générale (II) à oxyder.

c) Le diaphragme séparateur est constitué par un matériau poreux tel qu'une plaque, un manchon ou une bougie de verre fritté ou de porcelaine ou bien encore par une membrane échangeuse d'ions, de préférence une membrane échangeuse de cations. Il est particulièrement avantageux d'utiliser une membrane NAFION 125 (Marque déposée Dupont).

d) Le compartiment cathodique contient le même électrolyte que celui du compartiment anodique.

e) La cathode est constituée d'un matériau conducteur de l'électricité dont la nature n'est pas essentielle à la mise en œuvre du procédé et qui peut donc de ce fait être identique au matériau de l'anode ou être différent de celui-ci.

Selon une mise en œuvre préférée de l'invention, l'anode, la cathode et le diaphragme séparateur sont disposés selon des plans parallèles verticaux. En outre, plusieurs électrolyseurs élémentaires peuvent être associés à la manière de filtres-presses.

Le parcours de l'anolyte en circuit fermé peut être assuré par une pompe. Le circuit peut en outre comprendre des dispositifs annexes tels qu'échangeurs de température ou vases d'expansion ; un tel vase d'expansion permet en particulier d'alimenter l'anolyte en sulfure de formule générale (II) et permet également d'effectuer un soutirage pour l'extraction du sulfoxyde de formule générale (I).

Le catholyte peut également être soumis à une circulation. Selon un mode préférentiel de réalisation de l'invention, le circuit du catholyte est similaire à celui de l'anolyte, ce qui permet d'équilibrer les pressions de part et d'autre du diaphragme séparateur.

Selon un autre mode de mise en œuvre particulier de l'invention, on dispose des intercalaires dans les compartiments anodique et cathodique. Ces intercalaires servent à éviter, d'une part, les déformations de la membrane échangeuse d'ions et, d'autre part, les contacts de cette membrane avec les électrodes. Ils servent également à améliorer l'homogénéité de concentration de l'anolyte. Ils créent aussi des turbulences qui favorisent l'électrolyse. Ces intercalaires sont généralement fabriqués à partir de polymères synthétiques inertes chimiquement et non conducteurs de l'électricité. Ils peuvent être mis sous forme de fils entrelacés, entrecroisés, noués ou soudés (tissus, grilles, filets) ou bien encore sous forme de plaques munies de trous ou de rainures. En pratique ces intercalaires sont orientés selon des plans parallèles à ceux des électrodes et du diaphragme.

Selon une autre mise en œuvre de l'invention, la cellule peut être constituée simplement d'un récipient, parallélépipédique ou cylindrique, en une matière inerte vis-à-vis des constituants des électrolytes. Ce récipient contient l'électrode de travail dont la nature est la même que celle définie précédemment. La forme de cette électrode de travail est adaptée à la forme du récipient.

D'une façon générale, toute cellule électrolytique comportant une anode et une cathode séparées par un ou plusieurs diaphragmes assurant la conductibilité ionique est susceptible d'être employée, la disposition des éléments n'étant pas essentielle à la mise en œuvre du procédé.

Les produits obtenus par le procédé selon l'invention peuvent être purifiés par les méthodes physico-chimiques habituelles, notamment la cristallisation et la chromatographie.

Les produits de formule générale (II) peuvent être préparés par utilisation ou adaptation des méthodes décrites dans la demande de brevet européen publiée sous le numéro 0 046 417.

L'oxydation électrochimique indirecte utilisant un ion iodonium $I^+$ est connue par la publication de TATSUYA SHONO et Coll. [Tetrahedron Letters, (1979), 165-168]. Toutefois, rien dans cette publication ne permettait de penser que le procédé serait utilisable pour oxyder des sulfures de formule générale (II) et encore moins de les oxyder sélectivement.

Les produits de formule générale (I) obtenus par le procédé selon l'invention présentent des propriétés anti-hypertensives les rendant utiles comme médicaments destinés à traiter les hypertensions.

A des doses comprises entre 0,02 et 50 mg/kg p.o., ils abaissent la pression artérielle chez le rat spontanément hypertendu (rat SHR) de souche OKAMOTO-AOKI. L'utilisation du rat spontanément hypertendu pour l'étude des produits anti-hypertenseurs est décrite par J.L. ROBA, Lab. Anim. Sci., *26*, 305 (1976).

Leur dose létale ($DL_{50}$) chez la souris est généralement supérieure à 300 mg/kg p.o.

Les exemples suivants, donnés à titre non limitatif, montrent comment on peut mettre en pratique l'invention.

Exemple 1

Dans une cellule d'électrolyse de contenance 150 $cm^3$ comportant une électrode de travail constituée d'une grille de platine de 16 $cm^2$ de surface, une contre-électrode constituée d'une grille de platine de 4,5 $cm^2$ de surface et une électrode de référence au calomel saturée séparée de l'électrolyte par un gel d'agar-agar avec KCl, on introduit successivement 2 g de N-méthyl (pyridyl-3)-2 tétrahydrothiophènecarbothioamide-2, 67,5 $cm^3$ d'acétonitrile, 7,5 $cm^3$ d'eau permutée, 75 $cm^3$ de tampon phosphate d'ammonium aqueux [$NH_4H_2PO_4$ 0,1 M ; $(NH_4)_2HPO_4$ 0,1 M] à pH 7 et 0,4 g d'iodure de triéthyl n-propylammonium. Le mélange réactionnel, agité au moyen d'un barreau magnétique recouvert de polytétrafluoroéthylène, est désoxygéné par barbotage d'un courant d'azote. On fixe le potentiel de l'électrode de travail à + 0,8 v par rapport à l'électrode au calomel saturée (que nous notons : + 0,8 v/ECS). Après passage de 1 150 Coulombs, la solution s'échauffe par effet Joule et atteint la

température de 42 °C. On ramène alors le potentiel de l'électrode de travail à + 0,65 v/ECS de manière à conserver une température d'environ 45 °C. Le courant traversant la cellule se situe entre 400 et 500 mA. Au cours de l'électrolyse, on ajoute une solution aqueuse d'ammoniaque environ 5N afin de maintenir le pH à une valeur voisine ou supérieure à 7. On arrête l'électrolyse lorsque la quantité d'électricité qui a traversé la cellule est égale à 3 188 Coulombs, soit 3,93 Faradays par mole de produit à oxyder.

Le mélange réactionnel est alors concentré à sec sous pression réduite (20 mmg ; 2,7 kPa). Le résidu est extrait 2 fois avec 140 $cm^3$ au total d'éther éthylique ; les phases éthérées sont lavées avec 50 $cm^3$ d'eau distillée et éliminées. Les phases aqueuses sont réunies et extraites 20 fois avec 50 $cm^3$ à chaque fois d'acétate d'éthyle. Les extraits organiques sont réunis et séchés sur du sulfate de sodium. La solution est filtrée et évaporée à sec sous pression réduite (20 mmg ; 2,7 KPa) à 40 °C. On obtient ainsi un premier lot de 1 394 mg de N-méthyl(pyridyl-3)-2 tétrahydrothiophènecarbothioamide-2 oxyde-1 forme trans, sous forme d'un produit blanc fondant à 207 °C après recristallisation dans l'acétate d'éthyle.

[Rf = 0,24 ; chromatographie sur couche mince de gel de silice ; solvant : Acétate d'éthyle-méthanol (75-25 en volumes)].

La couche aqueuse provenant de l'extraction précédente à l'acétate d'éthyle est reprise et extraite par 3 fois 180 $cm^3$ au total de butanol. Les phases organiques sont réunies, séchées sur du sulfate de sodium, filtrées et concentrées à sec sous pression réduite (20 mm de mercure ; 2,7 kPa) à 40 °C. On obtient ainsi 450 mg d'un produit brut que l'on purifie par chromatographie préparative sur couche mince de gel de silice (éluant : acétate d'éthyle/méthanol 70/30 en volumes). Après grattage de la zone contenant le produit (repérée par UV), celui-ci est désorbé par un mélange d'acétate d'éthyle et de méthanol (50/50 en volumes). On obtient ainsi un deuxième lot de 155 mg de N-méthyl (pyridyl-3)-2 tétrahydrothiophènecarbothioamide-2 oxyde-1 forme trans fondant à 207 °C.

Le N-méthyl (pyridyl-3)-2 tétrahydrothiophènecarbothioamide-2 peut être préparé comme décrit dans la demande de brevet européen publiée sous le numéro 0 046 417.

## Exemple 2

Dans une cellule d'électrolyse de contenance 150 $cm^3$ comportant une électrode de travail constituée d'une grille de platine de 16 $cm^2$ de surface, une contre-électrode constituée d'une grille de platine de 8 $cm^2$ de surface et une électrode de référence au calomel saturée séparée de l'électrolyte par un gel d'agar-agar avec KCl, on introduit successivement 2 g de N-méthyl (pyridyl-3)-2 tétrahydrothiopyrannecarbothioamide-2, 67,5 $cm^3$ d'acétonitrile, 7,5 $cm^3$ d'eau permutée, 75 $cm^3$ de tampon phosphate aqueux d'ammonium [$(NH_4)_2HPO_4$ 0,1 M ; $NH_4H_2PO_4$ 0,1 M] à pH 7 et 0,25 g d'iodure d'ammonium.

Le mélange réactionnel, agité au moyen d'un barreau magnétique recouvert de polytétrafluoroéthylène, est désoxygéné par barbotage d'un courant d'azote. On fixe le potentiel de l'électrode de travail à + 0,8 v par rapport à l'électrode au calomel saturée (que nous notons : + 0,8 v/ECS). Le courant traversant la cellule décroît de 700 mA à 150 mA.

Au cours de l'électrolyse, on ajoute une solution aqueuse d'ammoniaque environ 5N afin de maintenir le pH à une valeur voisine ou supérieure à 7. On arrête l'électrolyse lorsque la quantité d'électricité qui a traversé la cellule est égale à 5 484 Coulombs, soit 7,15 Faradays par mole de produit à oxyder.

Le mélange réactionnel est alors concentré à sec sous pression réduite (20 mm de mercure ; 2,7 kPa). Le résidu est extrait 3 fois avec 50 $cm^3$ au total d'éther éthylique ; les phases éthérées sont lavées 2 fois avec 30 $cm^3$ d'eau distillée et éliminées. Les phases aqueuses sont réunies et extraites 10 fois par 300 $cm^3$ au total d'acétate d'éthyle pour éliminer les sous-produits non polaires. Les phases organiques sont lavées 5 fois avec 50 $cm^3$ d'eau distillée.

Les phases aqueuses sont réunies et extraites 3 fois par 150 $cm^3$ au total de butanol-1. Les phases organiques sont réunies, séchées sur du sulfate de sodium, filtrées et concentrées à sec sous pression réduite (20 mm de mercure ; 2,7 kPa) à 30 °C. On obtient ainsi 850 mg d'un produit brut que l'on purifie par chromatographie préparative sur couche mince de gel de silice (éluant : acétate d'éthyle/méthanol 70/30 en volumes). Après grattage de la zone contenant le produit (repérée par UV), celui-ci est désorbé par un mélange d'acétate d'éthyle et de méthanol (50/50 en volumes). On obtient ainsi 392 mg de N-méthyl (pyridyl-3)-2 tétrahydrothiopyrannecarbothioamide-2 oxyde-1 forme trans fondant à 228 °C.

[Rf = 0,24 ; chromatographie sur couche mince de gel de silice ; solvant : acétate d'éthyle-méthanol (75-25 en volumes)].

Le N-méthyl(pyridyl-3)-2 tétrahydrothiopyrannecarbothioamide-2 peut être préparé comme décrit dans la demande de brevet européen publiée sous le numéro 0 046 417.

## Revendications

1. Procédé de préparation de sulfoxydes de dérivés de la thioformamide de formule générale :

(dessin page 5)

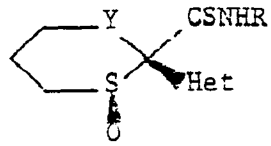

dans laquelle R représente un atome d'hydrogène ou un radical alcoyle contenant 1 à 4 atomes de carbone en chaîne droite ou ramifiée, Het représente un radical hétérocyclique à caractère aromatique contenant un ou deux atomes d'azote choisi parmi pyridyle-3 (éventuellement substitué par un radical alcoyle contenant 1 à 4 atomes de carbone en chaîne droite ou ramifiée ou par un atome d'halogène), quinolyle-3, pyridazinyle-4, pyrimidinyle-5, thiazolyle-5, thiéno [2,3-b] pyridyle-5 et thiéno [3,2-b] pyridyle-6 et Y représente une liaison de valence ou un radical méthylène, caractérisé en ce que l'on oxyde par voie électrochimique des produits de formule générale :

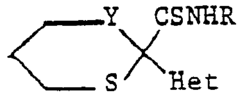

dans laquelle les symboles R, Het et Y sont définis comme ci-dessus en opérant dans un électrolyte à forte teneur en eau, à un pH compris entre 7 et 7,5 en présence d'un agent spécifique d'oxydation $X^+$ obtenu *in situ* par voie électrochimique à partir d'un halogénure $X^-$ en travaillant à un potentiel d'électrode imposé voisin du potentiel d'oxydation de $X^-$, puis isole le produit obtenu.

2. Procédé selon la revendication 1, caractérisé en ce que l'agent d'oxydation $X^+$ est obtenu à partir d'un halogénure d'ammonium.

3. Procédé selon la revendication 2, caractérisé en ce que l'halogénure d'ammonium est l'iodure d'ammonium.

4. Procédé selon la revendication 2, caractérisé en ce que l'halogénure d'ammonium est l'iodure de triéthyl n-propyl-ammonium.

5. Procédé selon la revendication 1, caractérisé en ce que le potentiel d'électrode imposé se situe entre 0,6 et 0,8 V par rapport à une électrode au calomel saturée.

6. Procédé selon la revendication 1, caractérisé en ce que l'électrolyte à forte teneur en eau contient un solvant organique miscible à l'eau.

7. Procédé selon la revendication 6, caractérisé en ce que le solvant organique miscible à l'eau est l'acétonitrile.

**Claims**

1. Process for the preparation of sulphoxides of thioformamide derivatives of general formula :

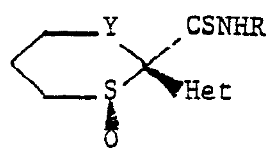

in which R denotes a hydrogen atom or a straight-chain or branched alkyl radical containing 1 to 4 carbon atoms, Het denotes a heterocyclic radical of an aromatic nature containing one or two nitrogen atoms which is chosen from 3-pyridyl (optionally substituted by a straight-chain or branched alkyl radical containing 1 to 4 carbon atoms or by a halogen atom), 3-quinolyl, 4-pyridazinyl, 5-pyrimidinyl, 5-thiazolyl, 5-thieno [2,3-b] pyridyl and 6-thieno [3,2-b] pyridyl, and Y denotes a valency bond or a methylene radical, characterized in that products of general formula :

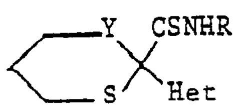

in which the symbols R, Het and Y are defined as above, are oxidized electrochemically, the operation being carried out in an electrolyte of high water content, at a pH of between 7 and 7.5, in the presence of a specific $X^+$ oxidizing agent obtained electrochemically *in situ* from a halide $X^-$ by working at an imposed electrode potential close to the oxidation potential of $X^-$, and the product obtained is then isolated.

2. Process according to Claim 1, characterized in that the oxidizing agent $X^+$ is obtained from an ammonium halide.

3. Process according to Claim 2, characterized in that the ammonium halide is ammonium iodide.

4. Process according to Claim 2, characterized in that the ammonium halide is triethyl-n-propylammonium iodide.

5. Process according to Claim 1, characterized in that the imposed electrode potential is between 0.6 and 0.8 V relative to a saturated calomel electrode.

6. Process according to Claim 1, characterized in that the electrolyte of high water content contains a water-miscible organic solvent.

7. Process according to Claim 6, characterized in that the water-miscible organic solvent is acetonitrile.

**Patentansprüche**

1. Verfahren zur Herstellung von Sulfoxiden von Derivaten des Thioformamids der allgemeinen Formel

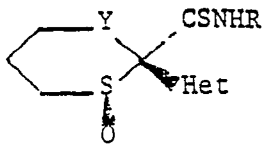

worin R ein Wasserstoffatom oder einen Alkylrest mit 1 bis 4 Hohlenstoffatomen in gerader oder verzweigter Kette bedeutet, Het einen heterocyclischen Rest mit aromatischem Charakter bedeutet, enthaltend ein oder zwei Stickstoffatome, ausgewählt unter Pyridyl-3 (gegebenenfalls substituiert durch einen Alkylrest, enthaltend 1 bis 4 Kohlenstoffatome in gerader oder verzweigter Kette, oder durch ein Halogenatom), Chinolyl-3, Pyridazinyl-4, Pyrimidinyl-5, Thiazolyl-5, Thieno [2,3-b] pyridyl-5 und Thieno [3,2-b] pyridyl-6, und Y eine Wertigkeitsbindung oder einen Methylenrest darstellt, dadurch gekennzeichnet, daß man auf elektrochemischem Wege Produkte der allgemeinen Formel :

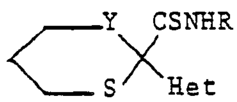

worin die Symbole R, Het und Y wie vorstehend definiert sind, oxidiert, wobei in einem Elektrolyten mit einem starken Gehalt an Wasser bei einem pH zwischen 7 und 7,5 in Gegenwart eines spezifischen Oxidationsmittels $X^+$ gearbeitet wird, das *in situ* auf elektrochemischem Wege, ausgehend von einem Halogenid $X^-$, erhalten wird, indem bei einem Elektrodenpotential gearbeitet wird, das nahe dem Oxidationspotential von $X^-$ gelegt ist, und das erhaltene Produkt dann isoliert wird.

2. Verfahren gemäß Anspruch 1, dadurch gekennzeichnet, daß das Oxidationsmittel $X^+$ ausgehend von einem Ammoniumhalogenid erhalten ist.

3. Verfahren gemäß Anspruch 2, dadurch gekennzeichnet, daß das Ammoniumhalogenid Ammoniumjodid ist.

4. Verfahren gemäß Anspruch 2, dadurch gekennzeichnet, daß das Ammoniumhalogenid Triethyl-n-propylammoniumjodid ist.

5. Verfahren gemäß Anspruch 1, dadurch gekennzeichnet, daß das angelegte Elektrodenpotential zwischen 0,6 und 0,8 V in bezug auf eine gesättigte Kalomelelektrode liegt.

6. Verfahren gemäß Anspruch 1, dadurch gekennzeichnet, daß der Elektrolyt mit einem starken Gehalt an Wasser ein mit Wasser mischbares, organisches Lösungsmittel enthält.

7. Verfahren gemäß Anspruch 6, dadurch gekennzeichnet, daß das mit Wasser mischbare, organische Lösungsmittel Acetonitril ist.